**Europäisches Patentamt**

**European Patent Office**

**Office européen des brevets**

(19)

(11) Veröffentlichungsnummer: **0 253 091 B1**

(12) # EUROPÄISCHE PATENTSCHRIFT

(45) Veröffentlichungstag der Patentschrift:
03.10.90

(21) Anmeldenummer: 87106975.3

(22) Anmeldetag: **14.05.87**

(51) Int. Cl.⁵: **C07C 31/20,** C07C 29/76,
C07C 29/86, C07C 29/80,
C07C 31/08

(54) Verfahren zum Abtrennen von niedermolekularen Alkoholen aus wässrigen Lösungen.

(30) Priorität: **15.07.86 DE 3623827**

(43) Veröffentlichungstag der Anmeldung:
**20.01.88 Patentblatt 88/3**

(45) Bekanntmachung des Hinweises auf die Patenterteilung:
**03.10.90 Patentblatt 90/40**

(84) Benannte Vertragsstaaten:
**CH DE FR GB IT LI NL**

(56) Entgegenhaltungen:
**DE-A- 3 037 736
DE-A- 3 112 603**

(73) Patentinhaber: **HÜLS AKTIENGESELLSCHAFT,
Patentabteilung / PB 15 - Postfach 13 20,
D-4370 Marl 1(DE)**

(72) Erfinder: **Sridhar, Srinivasan, Dr., Lehmbecker Pfad 52,
D-4370 Marl(DE)**

## Beschreibung

Die Erfindung betrifft ein Verfahren zur Abtrennung von niedermolekularen Alkoholen, insbesondere Butandiol-2,3 , aus deren wäßrigen Lösungen, die beispielsweise bei einer fermentativen Herstellung entstanden sind. Die Erfindung bezweckt, diese Abtrennung wirtschaftlicher zu gestalten.

Die Isolierung des bei einer Fermentation in kleinen Konzentrationen (5 - 10 Gew.-%) anfallenden Alkohols aus der Fermenterbrühe ist aufwendig und umständlich. Die Alkohole sind mit Wasser in allen Verhältnissen mischbar. Butandiol siedet bei einer wesentlich höheren Temperatur (180 °C/1 bar) als Wasser. Eine destillative Abtrennung erfordert in diesem Fall die unwirtschaftliche Verdampfung des gesamten Wassers. Der Einsatz von Wasserdampf zur Gewinnung von Butandiol wurde beschrieben; dabei entsteht zwar eine reine, aber verdünnte Lösung, die wieder destillativ entwässert werden muß (Ind. Eng. Chem. 38 (1946), S. 913). Ein weiterer umständlicher Weg ist die chemische Umsetzung des Diols zu Formal mittels Formaldehyd und die Umsetzung des Formals in Butandiol mittels Methanol (Ind. Eng. Chem. 37 (1945), S. 865).

Als Alternative zur Destillation bietet sich eine Extraktion an. Als Extraktionsmittel eignen sich beispielsweise Fettalkohole, wobei die niedermolekularen Alkohole, darunter insbesondere n-Butanol, ein hohes Aufnahmevermögen für das Diol haben (Ind. Eng. Chem. 37 (1945), S. 890). n-Butanol ist aber zum Teil löslich in Wasser und verbleibt im Raffinat, was eine zusätzliche Aufarbeitung erfordert. Daher wurden auch als Alternativen weniger lösliche Ester erwähnt, die aber ein entscheidend geringeres Lösevermögen für das Diol haben. Neuere Bestrebungen, insbesondere zur Isolierung von Ethanol aus Fermenterbrühen, zielen auf mehrfache Extraktion mit Lösemitteln, z. B. n-Hexanol und n-Dodekan, oder auf die Bereitstellung hochmolekularer, im Wasser wenig löslicher Extraktionsmittel, die aber wie die Fettalkohole auch Wasser aufnehmen (DE-OS-31 12 603). Es werden Polyoxyalkohole, Polyoxyalkanole und Polyoxyalkanpolyole erwähnt. Auch diese Lösemittel weisen kein mit Butanol und Hexanol vergleichbares Aufnahmevermögen auf, wie bereits am Beispiel von Ethanol als Wertstoff nachgewiesen wurde. Die starke Emulgatorwirkung solcher hochmolekularer Mittel sowie geringe Dichteunterschiede zwischen der organischen und wäßrigen Phase (insbesondere bei Butandiol) erschweren die Extraktion erheblich. Das Lösemittel im Extrakt kann durch Destillation oder Umkehrosmose vom gelösten Wasser und Wertstoff befreit werden, aber dabei verbleiben im Lösemittel auch unerwünschte systemeigene Hochsieder wie das Dimere von Acetoin im Falle von Butandiol. Der erforderliche Extraktionsaufwand ist größer als bei Butanol bzw. Hexanol und das Raffinat enthält noch Alkoholreste und Spuren von Lösemitteln, die zu Verlusten und Entsorgungsproblemen führen. Auch der Verfügbarkeit und den Herstellkosten solcher Lösemittel muß Rechnung getragen werden.

Nach dem Stand der Technik zielen alle Bestrebungen darauf, unter Verzicht auf das gute Aufnahmevermögen eines verfügbaren niedermolekularen Extraktionsmittels Verbindungen höheren Molekulargewichtes zur Extraktion heranzuziehen, die weniger wasserlöslich sind. Damit besteht mehrfach ein Vorurteil gegen die Verwendung niedermolekularer Stoffe als Extraktionsmittel. Bei der Umkehrosmose nach DE-OS 31 12 603 wird ein hochmolekulares Extraktionsmittel vom Wertstoff und Wasser getrennt.

Damit stellt sich die Aufgabe, ein Extraktionsverfahren derart zu gestalten, daß der Einsatz von niedermolekularen Extraktionsmitteln vorteilhaft durchführbar wird. Das Verfahren soll nicht nur eine extraktive Rückgewinnung der eingesetzten Stoffe, sondern auch eine sinnvolle Ausschleusung sonstiger Stoffe und des Wassers ermöglichen.

Diese Aufgabe wird erfindungsgemäß gelöst durch ein Verfahren mit folgenden kennzeichnenden Merkmalen:
- Aufkonzentrieren der wäßrigen Lösung durch Umkehrosmose und Ausschleusen des Wassers ausschließlich durch die Umkehrosmose,
- Extrahieren des Wertstoffes mittels eines niedermolekularen Extraktionsmittels,
- Destillieren des Extraktstromes und Ausschleusen des wasserfreien Wertstoffes oder seines Azeotrops aus der Destillationskolonne,
- Rückführen des Destillatstromes oder des Sumpfstromes in die Extraktionskolonne,
- Rückführen des Raffinatstromes aus der Extraktionskolonne in den Zulaufstrom der Umkehrosmose-Einheit.

Die wäßrige Lösung des Wertstoffes, die der Umkehrosmose unterworfen wird, muß frei sein von Schwebeteilchen. Falls eine Fermenterbrühe nach dem erfindungsgemäßen Verfahren aufgearbeitet werden soll, wird diese beispielsweise durch Mikrofiltration von Schwebeteilchen befreit.

Die Umkehrosmose kann je nach der Membranqualität und Erfordernissen hinsichtlich der zulässigen Stoffverluste im Permeat, ein oder mehrstufig erfolgen.

Liegen wertvolle höhermolekulare Stoffe in der Fermenterbrühe in bedeutenden Anteilen vor, so können sie vor dem Aufkonzentrierungsschritt durch eine weitere vorgeschaltete Umkehrosmose mit entsprechend gewählter Membran oder ein anderes Membranverfahren, z. B. Ultrafiltration, vorbehandelt werden: Das Retentat dieser vorgeschalteten Stufe enthält die höhermolekularen Stoffe und wird in die Fermentation zurückgeführt. Das Permeat dieser vorgeschalteten Stufe besteht aus Wasser und dem Wertstoff. Erst danach erfolgt die Aufkonzentrierung des Wertstoffes durch Umkehrosmose unter Zumischen von Raffinat aus der Extraktion. Damit wird eine Kontamination des Fermentationsschrittes mit dem Extraktionsmittel und anderen für die Fermentation schädlichen Stoffen vermieden.

Die Besonderheit des erfindungsgemäßigen Verfahrens liegt darin, daß die eindeutig vorteilhafte Eigenschaft von niedermolekularen Stoffen als Extraktionsmittel brauchbar gemacht wird, indem man die Extraktion des Wertstoffes mit dessen Aufkonzentrieren durch Umkehrosmose koppelt. Das erfindungsgemäße Verfahren bewirkt die Abtrennung sowohl des Wertstoffs als auch des Extraktionsmittels gemeinsam vom Wasser und zwar angesichts der Bestrebungen, Membrane herzustellen, die nie dermolekulare Stoffe wie Ethanol fast vollständig zurückhalten, jedoch Wasser durchlassen (Biotechnology and Bioengineering 27 (1985), S. 1081). Die Umkehrosmose löst als ein einzelner Verfahrensschritt gleichzeitig die folgenden Aufgaben:

Aufkonzentrieren des Wertstoffes, wodurch die darauffolgenden Schritte der Extraktion und Destillation erheblich wirtschaftlicher werden.

Ausschleusen des gesamten Wassers.

Das erfindungsgemäße Verfahren hat folgende Vorteile:

- Das gesamte Wasser der Ausgangslösung verläßt den Prozeß ausschließlich über den Permeatstrom der Umkehrosmose-Einheit, d. h. ohne thermische Trennung von Wertstoff und Wasser.

- Reste des Wertstoffes aus der Destillation und Reste des Extraktionsmittels aus dem Raffinat der Extraktion werden in den Zulaufstrom der Umkehrosmose zurückgeführt und gehen damit nicht verloren. Das Permeat der Umkehrosmose enthält weniger als 0,5 Gewichtsprozent an Wertstoff.

- Das Extraktionsverfahren ist einstufig. Eine zweite Extraktionsstufe zum Entfernen des Extraktionsmittels aus der ersten Extraktionsstufe ist nicht erforderlich.

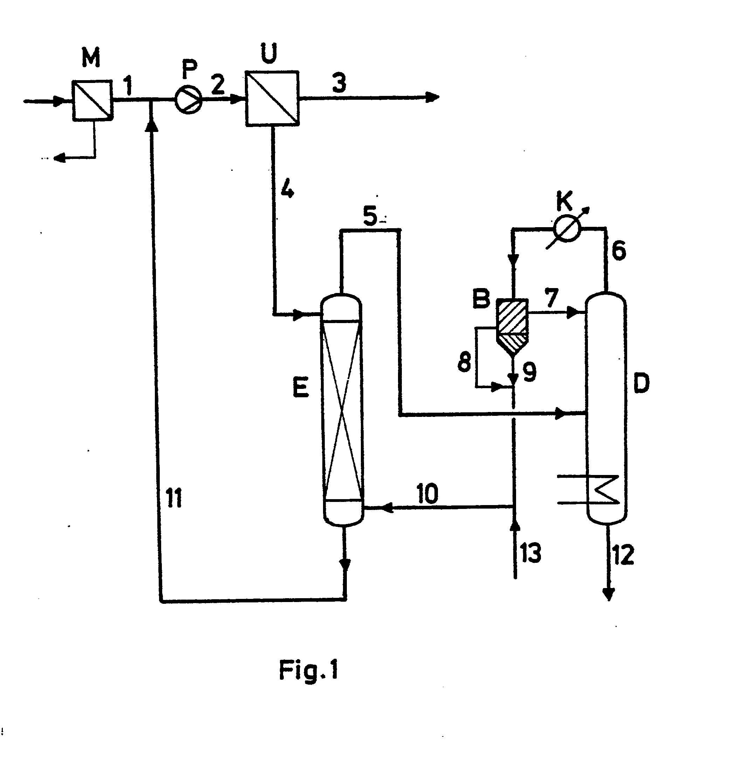

In den Figuren 1 bis 3 sind zur Durchführung des erfindungsgemäßen Verfahrens geeignete Vorrichtungen dargestellt; das Verfahren ist jedoch nicht auf diese Vorrichtungen beschränkt. Die Bezugszeichen bedeuten:

M: Mikrofiltrationsgerät
P: Hochdruckpumpe
U, $U_1$, $U_2$ : Umkehrosmose-Einheiten
E: Extraktionskolonne
D, $D_1$, $D_2$, $D_3$: Destillationskolonnen
K, $K_1$, $K_2$, $K_3$: Kondensatoren
B: Trennbehälter

Weitere bekannte und zur Durchführung des erfindungsgemäßen Verfahrens nebensächliche Geräte sind nicht dargestellt.

Das erfindungsgemäße Verfahren wird an Hand der folgenden Beispiele weiter erläutert, ohne hierauf beschränkt zu sein.

Beispiel 1: Abtrennung von Butandiol-2,3 und Acetoin als Wertstoff mit n-Butanol als Extraktionsmittel

Die benutzte Vorrichtung ist in Fig. 1 angegeben, die Stoffströme sind in Tab. 1 zusammengestellt. Der Wertstoff liegt in einer Fermenterbrühe gelöst vor, die im wesentlichen aus Wasser, Butandiol-2,3, geringen Mengen Acetoin und Biomasse besteht. Die Fermenterbrühe wird beispielsweise durch eine Mikrofiltration (M) von Schwebeteilchen über 0,2 μm befreit. Das Retentat der Mikrofiltration wird in die Fermentation zurückgeführt, während das Permeat der Mikrofiltration (Strom 1) der Aufarbeitung zur Verfügung steht. Diese Ausgangslösung wird mit dem das Extraktionsmittel n-Butanol enthaltenden Raffinatstrom (11) gemischt, mittels einer Hochdruckpumpe (P) über eine Umkehrosmose-Einheit (U) geschickt (Strom 2) und auf 13 Gew.-% Butandiol-2,3 eingeengt. Hierfür wird beispielsweise die Membran HR 98 von DDS/Filmtech (DDS heißt: De Dansk Sukkerfabrikker) eingesetzt. Der Betriebsdruck beträgt 7,5 MPa und die Temperatur 30 °C. Der Permeatstrom (3) der Umkehrosmose-Einheit besteht aus fast reinem Wasser, das Spuren des Wertstoffes und Spuren des Extraktionsmittels enthält.

Der Retentatstrom (4) der Umkehrosmose-Einheit, der im wesentlichen aus Wasser, Wertstoff und Extraktionsmittel besteht, gelangt in eine Extraktionskolonne (E), die beispielsweise mit Siebböden versehen ist. Die Extraktion erfolgt bei Raumtemperatur und Normaldruck, z. B. über 6 theoretische Stufen im Gegenstrom. Der Extraktstrom (5) wird in der Destillationskolonne (D) destilliert, wobei n-Butanol und Wasser vollständig über Kopf abgenommen werden, während Butandiol-2,3 sowie Teile des Acetoin als Wertstoff im wasserfreien Sumpf verbleiben.

Die Destillation in der Kolonne (D) erfolgt beispielsweise bei einem molaren Rücklaufverhältnis 5 und 30 theoretischen Trennstufen und 200 hPa. Die Kopftemperatur beträgt 62 °C. Das Kondensat der Dämpfe (6) zerfällt im Trennbehälter (B) bei Raumtemperatur in zwei Phasen, wobei ein Teil der oberen Phase, die überwiegend aus n-Butanol besteht, als Rücklauf (7) in die Kolonne (D) zurückgeführt wird. Das Destillat, bestehend aus beiden Phasen, nämlich aus der unteren Phase aus n-Butanol-haltigem Wasser (Strom 9) und dem anderen Teil der oberen Phase aus wasserhaltigem n-Butanol (Strom 8), wird in die Extraktion zurückgeführt. Die geringen Verluste an n-Butanol in Strom (3) können durch Einspeisen von n-Butanol (Strom 13) ersetzt werden.

Der wasserfreie Sumpfstrom (12), der Butandiol-2,3 und Acetoin als Wertstoff enthält, wird bekannterweise in einer weiteren nicht dargestellten Kolonne destillativ getrennt, beispielsweise bei 70 °C und

3

80 hPa am Kopf beim Rücklaufverhältnis 2 und 22 theoretischen Trennstufen, mit reinem Acetoin als Kopfprodukt und reinem Butandiol-2,3 als Sumpfprodukt.

Falls die Ströme (8) und (9) nicht vereinigt werden und nicht über Strom (10) in die Extraktionskolonne (E) zurückgeführt werden, kann Strom (8) in den unteren Teil der Kolonne (E) zurückgeführt werden und Strom (9) in einen anderen Teil der Kolonne (E) oder direkt in den Strom (11).

Beispiel 2: Abtrennung von Butandiol-2,3 als Wertstoff mit n-Hexanol als Extraktionsmittel

Die benutzte Vorrichtung ist in Fig. 1 angegeben, die Stoffströme sind in Tab. 2 zusammengestellt. Der Wertstoff liegt in einer Fermenterbrühe gelöst vor, die derjenigen des Beispiels 1 entspricht, die aber frei von Acetoin und Salzen ist. Anstatt n-Butanol wird n-Hexanol als Extraktionsmittel eingesetzt.

Da die gegenseitige Löslichkeit zwischen Wasser und n-Hexanol wesentlich geringer ist als die zwischen Wasser und n-Butanol, sind die Raffinat- bzw. Extraktströme entsprechend ärmer an n-Hexanol bzw. Wasser. Der Beitrag von n-Hexanol im Raffinatstrom zum osmotischen Druck wird geringer. Auch die Retention an der Membran der Umkehrosmose-Einheit wird besser. Andererseits ist der Extraktionsaufwand größer, beispielsweise ist das Gewichtsverhältnis n-Hexanol/Wasser gleich 3 bei 6 theoretischen Trennstufen.

In Kolonne (D) wird n-Hexanol (und die geringe Wassermenge) als Kopfprodukt von Butandiol-2,3 abgetrennt und zwar bei den Kopfbedingungen 86 °C, 200 hPa, beim Rücklaufverhältnis 2 und 24 theoretischen Trennstufen. Als Rücklauf wird ein Teil des gesamten Destillats ohne Absetzen im Behälter (B) direkt zurückgeführt, der andere Teil des Destillatstromes wird über Strom (10) in die Extraktionskolonne (E) zurückgeführt. In diesem Falle wird der Strom (6) in die Ströme (7) und (10) - letzterer nach Abzug von Strom (13) - aufgeteilt.

Das wasserfreie Butandiol-2,3 gelangt als Strom (12) zur Weiterreinigung oder kann bekannterweise in der Kolonne (D), bei entsprechenden Trennstufen, selbst als Seitenstrom abgetrennt werden; durch Zusatz eines systemfremden Hochsieders können dann evtl. systemeigene Hochsieder als Sumpfprodukt abgeführt werden.

Beispiel 3: Abtrennung von Butandiol-2,3 und Acetoin als Wertstoff mit n-Hexanol als Extraktionsmittel und Trennen von Butandiol-2,3 und Acetoin

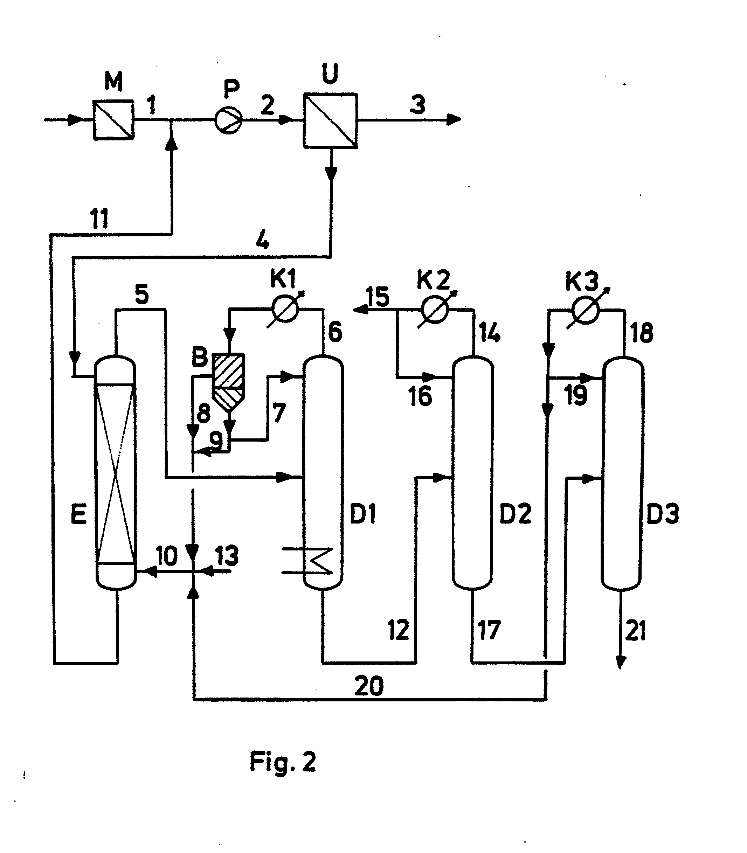

Die benutzte Vorrichtung ist in Fig. 2 angegeben, die Stoffströme sind in Tab. 3 zusammengestellt. Der Wertstoff liegt in einer Fermenterbrühe gelöst vor, die derjenigen des Beispiels 1 entspricht.

Die Fermenterbrühe enthält also Butandiol-2,3 und Acetoin als Wertstoff (wie im Beispiel 1). Mit n-Hexanol als Extraktionsmittel werden Butandiol-2,3 und Acetoin extrahiert (wie im Beispiel 2). Die Kolonne (D1) dient (analog zu Kolonne (D) in Beispiel 2) zur Entwässerung:

Bei 60 °C und 200 hPa am Kopf, molarem Rücklaufverhältnis 1 und 16 theoretischen Trennstufen wird das n-Hexanol/Wasser-Azeotrop in den Trennbehälter (B) geführt, und das Destillat gelangt in den Strom (10).

Das Sumpfprodukt der Kolonne (D1) wird als Strom (12) in der nächsten Kolonne (D2) bei 75 °C und 100 hPa am Kopf und beim Rücklaufverhältnis 7 und 30 theoretischen Trennstufen destilliert. Das Acetoin wird als Strom (15) entnommen und teilweise als Strom (16) in die Kolonne (D2) zurückgeführt. Das Gemisch aus n-Hexanol und Butandiol-2,3 (Strom 17) wird in der Kolonne (D3) bei 112 °C und 200 hPa am Kopf (Rücklaufverhältnis 1 und 30 theoretische Trennstufen) aufgetrennt. Ein Teil von n-Hexanol gelangt als Strom (20) über den Strom (10) in die Extraktionsstufe (E). Das Butandiol-2,3 verbleibt im Sumpf, aus dem es als Strom (21) entnommen werden kann, oder kann, wie im Beispiel 2 beschrieben, als Seitenstrom abgetrennt werden.

Beispiel 4: Abtrennung von Ethanol als Wertstoff mit n-Hexanol als Extraktionsmittel

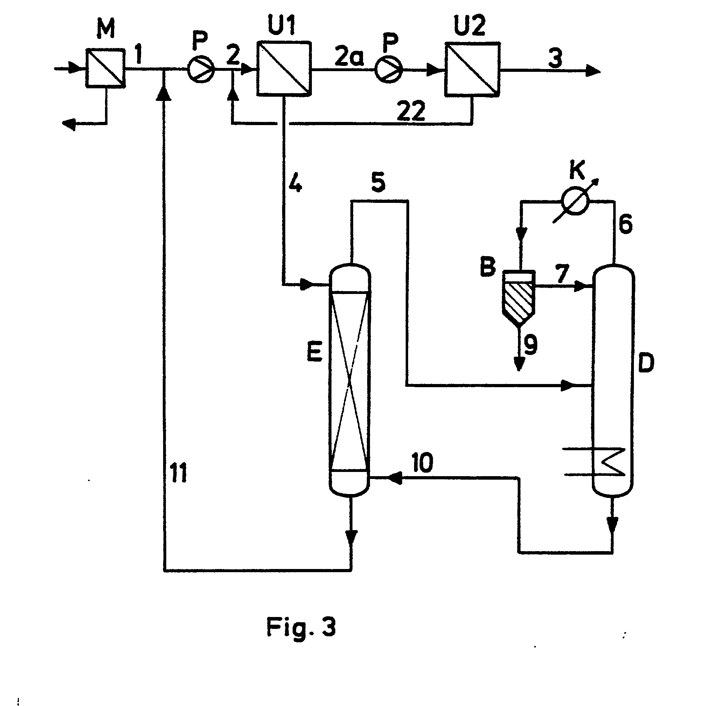

Die benutzte Vorrichtung ist in Fig. 3 angegeben, die Stoffströme sind in Tab. 4 zusammengestellt. Der Wertstoff liegt wiederum in einer Fermenterbrühe gelöst vor.

Die Fermenterbrühe enthält 5 Gew.-% Ethanol. Um den Rückhalt von Ethanol durch Umkehrosmose besonders wirksam zu gestalten, erfolgt die Umkehrosmose zweistufig in den Einheiten (U1) und (U2), jeweils bei 6 MPa und 30 °C. Als Membran wird beispielsweise der Typ PEC-1000 der Fa. Toray verwendet. Das Retentat der zweiten Umkehrosmose-Stufe wird als Strom (22) in den Zulaufstrom (2) der ersten Umkehrosmose-Stufe zurückgeführt. Die Extraktion erfolgt mit n-Hexanol als Extraktionsmittel.

Der Wertstoff Ethanol (mit azeotropem Anteil an Wasser) fällt am Kopf der Kolonne (D) an; er wird als Strom (9) entnommen und kann gegebenenfalls in bekannter Weise entwässert werden. Die Kopfbedingungen sind 78 °C bei 1 000 hPa beim Rücklaufverhältnis 5 und 26 theoretischen Trennstufen.

Der wasserhaltige n-Hexanolstrom (10) wird in die Extraktionskolonne (E) zurückgeführt. Dieser Strom wird in einer weiteren nicht dargestellten Kolonne periodisch destillativ von sonstigen Komponenten befreit, und reines n-Hexanol wird periodisch in den Strom (11) eingespeist.

Beispiel 5: Abtrennung von Ethanol als Wertstoff mit n-Butanol als Extraktionsmittel

Die benutzte Vorrichtung ist in Fig. 3 angegeben.
Das Beispiel 4 wird wiederholt, jedoch wird an Stelle von n-Hexanol jetzt n-Butanol als Extraktionsmittel eingesetzt.
Das Ergebnis ist ähnlich zu dem Ergebnis von Beispiel 4.

Tabelle 1: Stoffströme zu Beispiel 1; Angaben in kg/h

| Strom | 1 | 2 | 3 | 4 | 5 | 6 | 7 | 8 | 9 | 10 | 11 | 12 | 13 |
|---|---|---|---|---|---|---|---|---|---|---|---|---|---|
| Stoff | | | | | | | | | | | | | |
| n- Butanol | - | 5,64 | 0,3 | 5,34 | 161,9 | 1117,98 | 956,08 | 160,66 | 1,24 | 162,2 | 5,64 | - | 0,3 |
| Wasser | 194,3 | 275,4 | 194,3 | 81,1 | 54,4 | 290,74 | 236,34 | 39,67 | 14,73 | 54,4 | 81,1 | - | - |
| Acetoin | 1,56 | 1,66 | 0,03 | 1,63 | 2,53 | 1,0 | - | 1,0 | - | 1,0 | 0,10 | 1,53 | - |
| Butandiol-2,3 | 12,5 | 13,31 | 0,3 | 13,01 | 12,2 | - | - | - | - | - | 0,81 | 12,2 | - |
| Summe | 208,36 | 296,01 | 194,93 | 101,08 | 231,03 | 1409,72 | 1192,42 | 201,33 | 15,97 | 217,6 | 87,65 | 13,73 | 0,3 |

EP 0 253 091 B1

Tabelle 2: Stoffströme zu Beispiel 2; Angaben in kg/h

| Strom | 1 | 2 | 3 | 4 | 5 | 6 | 7 | 8 | 9 | 10 | 11 | 12 | 13 |
|---|---|---|---|---|---|---|---|---|---|---|---|---|---|
| Stoff | | | | | | | | entfällt | | | | | |
| n-Hexanol | - | 0,37 | 0,03 | 0,34 | 181,38 | 544,14 | 362,76 | | | 181,41 | 0,37 | - | 0,03 |
| Wasser | 194,3 | 254,76 | 194,3 | 60,46 | 8,07 | 24,21 | 16,14 | | | 8,07 | 60,46 | - | - |
| Butandiol-2,3 | 12,5 | 13,31 | 0,3 | 13,01 | 12,2 | - | - | | | - | 0,81 | 12,2 | - |
| Summe | 206,8 | 268,44 | 194,63 | 73,81 | 201,65 | 568,35 | 378,9 | | | 189,48 | 61,64 | 12,2 | 0,03 |

EP 0 253 091 B1

Tabelle 3: Stoffströme zu Beispiel 3; Angaben in kg/h

| Strom | 1 | 2 | 3 | 4 | 5 | 6 | 7 | 8 | 9 | 10 | 11 |
|---|---|---|---|---|---|---|---|---|---|---|---|
| **Stoff** | | | | | | | | | | | |
| n-Hexanol | - | 0,37 | 0,03 | 0,34 | 181,35 | 2,11 | 0,05 | 1,96 | 0,1 | 181,38 | 0,37 |
| Wasser | 194,3 | 254,76 | 194,3 | 60,46 | 8,07 | 16,49 | 8,42 | 0,12 | 7,95 | 8,07 | 60,46 |
| Acetoin | 1,56 | 1,66 | 0,03 | 1,63 | 1,53 | - | - | - | - | - | 0,10 |
| Butandiol-2,3 | 12,5 | 13,31 | 0,3 | 13,01 | 12,2 | - | - | - | - | - | 0,81 |
| **Summe** | 208,36 | 270,1 | 194,66 | 75,44 | 203,15 | 18,60 | 8,47 | 2,09 | 8,05 | 189,45 | 61,74 |

| Strom | 12 | 13 | 14 | 15 | 16 | 17 | 18 | 19 | 20 | 21 |
|---|---|---|---|---|---|---|---|---|---|---|
| **Stoff** | | | | | | | | | | |
| n-Hexanol | 179,29 | 0,03 | - | - | - | 179,29 | 358,58 | 179,29 | 179,29 | - |
| Wasser | - | - | - | - | - | - | - | - | - | - |
| Acetoin | 1,53 | - | 12,24 | 1,53 | 10,71 | - | - | - | - | - |
| Butandiol-2,3 | 12,2 | - | - | - | - | 12,2 | - | - | - | 12,2 |
| **Summe** | 193,02 | 0,03 | 12,24 | 1,53 | 10,71 | 191,49 | 358,58 | 179,29 | 179,29 | 12,2 |

EP 0 253 091 B1

Tabelle 4: Stoffströme zu Beispiel 4; Angaben in kg/h

| Strom / Stoff | 1 | 2 | 2a | 3 | 4 | 5 | 6 | 7 | 8 | 9 | 10 | 11 | 22 |
|---|---|---|---|---|---|---|---|---|---|---|---|---|---|
| | | | | | | | | | entfällt | | | | |
| n-Hexanol | - | 0,34 | - | - | 0,34 | 170,1 | - | - | | - | 170,1 | 0,34 | - |
| Wasser | 90,0 | 146,28 | 134,58 | 89,58 | 56,70 | 9,42 | 2,52 | 2,1 | | 0,42 | 9,0 | 56,28 | 45,0 |
| Ethanol | 10,0 | 10,1 | 1,0 | 0,1 | 10,0 | 9,9 | 59,4 | 49,5 | | 9,9 | - | 0,1 | 0,9 |
| Summe | 100,0 | 156,72 | 135,58 | 89,68 | 67,04 | 189,42 | 61,92 | 51,6 | | 10,32 | 179,1 | 56,72 | 45,9 |

EP 0 253 091 B1

**Patentansprüche**

1. Verfahren zum Abtrennen von niedermolekularen Alkoholen als Wertstoffe aus einer wäßrigen Lösung gekennzeichnet durch,
- Aufkonzentrieren der wäßrigen Lösung durch Umkehrosmose und Ausschleusen des Wassers ausschließlich durch die Umkehrosmose,
- Extrahieren des Wertstoffes mittels eines niedermolekularen Extraktionsmittels,
- Destillieren des Extraktstromes und Ausschleusen des wasserfreien Wertstoffes oder seines wäßrigen Azeotrops aus der Destillationskolonne,
- Rückführen des Destillatstromes oder des Sumpfstromes in die Extraktionskolonne,
- Rückführen des Raffinatstromes aus der Extraktionskolonne in den Zulaufstrom der Umkehrosmose-Einheit.

2. Verfahren nach Anspruch 1, dadurch gekennzeichnet,
- daß aliphatische Alkohole mit 2 bis 4 C-Atomen als Wertstoff aus einer Fermenterbrühe abgetrennt werden.

3. Verfahren nach den Ansprüchen 1 und 2, dadurch gekennzeichnet,
- daß aliphatische Alkohole mit 4 bis 6 C-Atomen als Extraktionsmittel eingesetzt werden.

4. Verfahren nach den Ansprüchen 1 bis 3, dadurch gekennzeichnet,
- daß Butandiol-2,3 als Wertstoff unter Einsatz von n-Butanol als Extraktionsmittel abgetrennt wird.

5. Verfahren nach den Ansprüchen 1 bis 3, dadurch gekennzeichnet,
- daß Butandiol-2,3 als Wertstoff unter Einsatz von n-Hexanol als Extraktionsmittel abgetrennt wird.

6. Verfahren nach den Ansprüchen 1 bis 3, dadurch gekennzeichnet,
- daß Ethanol als Wertstoff unter Einsatz von n-Butanol als Extraktionsmittel abgetrennt wird.

7. Verfahren nach den Ansprüchen 1 bis 3, dadurch gekennzeichnet,
- daß Ethanol als Wertstoff unter Einsatz von n-Hexanol als Extraktionsmittel abgetrennt wird.

8. Verfahren nach den Ansprüchen 1 bis 7, dadurch gekennzeichnet,
- daß man für die Umkehrosmose mehrere, vorzugsweise zwei Stufen einsetzt, wobei das Retentat der zweiten Stufe in den Zulaufstrom der ersten Stufe zurückgeführt wird.

9. Verfahren nach dem Anspruch 1, dadurch gekennzeichnet,
- daß die Umkehrosmose bei 20 bis 60 °C, vorzugsweise 30 bis 40 °C und 5 bis 10 MPa, vorzugsweise 6 bis 8 MPa erfolgt.

10. Verfahren nach den Ansprüchen 1 bis 9, dadurch gekennzeichnet,
- daß der Wertstoff durch weitere Destillation aus dem Sumpfstrom oder dem Kopfstrom der Destillationskolonne in reiner Form dargestellt wird.

**Claims**

1. A process for the separation of low molecular weight alcohols as desired substances from an aqueous solution, characterised by
- concentration of the aqueous solution by reverse osmosis and removal of the water exclusively by reverse osmosis,
- extraction of the desired substance using a low molecular weight extractant,
- distillation of the extract stream and removal of the anhydrous desired substance or the aqueous azeotrope thereof from the distillation column,
- recycling of the distillate stream or of the bottom stream into the extraction column,
- recycling of the refined product stream from the extraction column into the feed stream of the reverse osmosis unit.

2. A process according to claim 1, characterised in that aliphatic alcohols having 2 to 4 carbon atoms are separated as the desired substance from a fermentation liquor.

3. A process according to either of claims 1 and 2, characterised in that aliphatic alcohols having 4 to 6 carbon atoms are used as extractants.

4. A process according to any of claims 1 to 3, characterised in that 2,3-butanediol is separated off as the desired substance using n-butanol as the extractant.

5. A process according to any of claims 1 to 3, characterised in that 2,3-butanediol is separated off as the desired substance using n-hexanol as the extractant.

6. A process according to any of claims 1 to 3, characterised in that ethanol is separated off as the desired substance using n-butanol as the extractant.

7. A process according to any of claims 1 to 3, characterised in that ethanol is separated off as the desired substance using n-hexanol as the extractant.

8. A process according to any of claims 1 to 7, characterised in that the reverse osmosis is carried out in a plurality of steps, preferably two, the retained product of the second step being recycled into the feed stream of the first step.

9. A process according to claim 1, characterised in that the reverse osmosis is carried out at 20 to 60°C, preferably 30 to 40°C, and 5 to 10 MPa, preferably 6 to 8 MPa.

10. A process according to any of claims 1 to 9, characterised in that the desired substance is pre-

EP 0 253 091 B1

pared in pure form by further distillation from the bottom stream or the head stream of the distillation column.

**Revendications**

1. Procédé pour séparer à partir d'une solution aqueuse des alcools de bas poids moléculaire servant de matières de valeur, caractérisé par:
   - La concentration de la solution aqueuse par osmose d'inversion et expulsion de l'eau exclusivement par l'osmose d'inversion,
   - L'extraction de la matière de valeur à l'aide d'un agent d'extraction de bas poids moléculaire,
   - La distillation du courant d'extrait et expulsion de la matière de valeur anhydre ou de son azéotrope aqueux de la colonne de distillation,
   - Le renvoi dans la colonne d'extraction du courant de distillat ou du courant du produit de queue,
   - Le renvoi du courant de raffinat de la colonne d'extraction dans le courant d'alimentation de l'unité d'osmose d'inversion.

2. Procédé selon la revendication 1, caractérisé par le fait que les alcools aliphatiques comportant de 2 à 4 atomes de carbone sont séparés comme matière de valeur à partir d'une bouillie de fermenteur.

3. Procédé selon les revendications 1 et 2, caractérisé par le fait que les alcools aliphatiques comportant de 4 à 6 atomes de carbone sont utilisés en tant qu'agents d'extraction.

4. Procédé selon les revendications 1 à 3, caractérisé par le fait que le butane-diol-(2,3) est séparé comme matière de valeur en utilisant du n-butanol en tant qu'agent d'extraction.

5. Procédé selon les revendications 1 à 3, caractérisé par le fait que le butane-diol-(2,3) est séparé comme matière de valeur en utilisant du n-hexanol en tant qu'agent d'extraction.

6. Procédé selon les revendications 1 à 3, caractérisé par le fait que l'éthanol est séparé comme matière de valeur en utilisant du n-butanol en tant qu'agent d'extraction.

7. Procédé selon les revendications 1 à 3, caractérisé par le fait que l'éthanol est séparé comme matière de valeur en utilisant du n-hexanol en tant qu'agent d'extraction.

8. Procédé selon les revendications 1 à 7, caractérisé par le fait que l'on utilise plusieurs stades pour l'osmose d'inversion, de préférence deux stades, le produit de rétention du second stade étant renvoyé dans le courant d'alimentation du premier stade.

9. Procédé selon la revendication 1, caractérisé par le fait que l'osmose d'inversion est effectuée à une température de 20 à 60°C, de préférence de 30 à 40°C, et sous une pression de 5 à 10 MPa, de préférence de 6 à 8 MPa.

10. Procédé selon les revendications 1 à 9, caractérisé par le fait que la matière de valeur est préparée sous forme pure par une autre distillation, à partir du courant du produit de queue ou du courant sortant à la tête de la colonne de distillation.

Fig.1

Fig. 2

EP 0 253 091 B1

Fig. 3